# EUROPEAN PATENT SPECIFICATION

(11) **EP 3 086 794 B1**
(45) Date of publication and mention of the grant of the patent: **08.01.2020**
(21) Application number: 14723881.0
(22) Date of filing: 28.03.2014
(51) Int. Cl.: A61K 31/704, C12P 19/56

(54) **PROCESS FOR THE CONVERSION OF COLCHICINOIDS TO THEIR 3-GLYCOSYLATED DERIVATIVES VIA THEIR RESPECTIVE 3-DEMETHYL ANALOGUES**
VERFAHREN ZUR UMWANDLUNG VON COLCHICINOIDEN IN IHRE 3-GLYCOSYLIERTEN DERIVATE ÜBER IHRE JEWEILIGEN 3-DEMETHYLANALOGA
PROCÉDÉ POUR LA CONVERSION DE COLCHICINOÏDES EN LEURS DÉRIVÉS 3-GLYCOSYLÉS PAR LEUR ANALOGUES 3-DÉMÉTHYL CORRESPONDANTS

(30) Priority: 23.12.2013 IN 6035CH2013
(43) Date of publication of application: 02.11.2016
(73) Proprietor: Alkaloids Corporation, Kolkata 700001 (IN)
(72) Inventor: KRISHNAMURTHI, Prabakaran, Kolkata 700001 (IN); KARNANI, Manish Kumar, Kolkata 700001 (IN)
(74) Representative: Hamm&Wittkopp Patentanwälte PartmbB
(86) International application number: PCT/IB2014/060093
(87) International publication number: WO 2015/097567

(56) References cited:
- WO-A1-98/15642
- WO-A1-2011/112161
- WO-A2-2012/038982
- SEMANTI RAY ET AL: "From Space to Earth: Bacillus aryabhattai found in the Indian sub-continent", BIOSCIENCE DISCOVERY, vol. 3, no. 1, January 2012 (2012-01), pages 138-145, XP002728213,
- DUBEY K K ET AL: "Production of demethylated colchicine through microbial transformation and scale-up process development", PROCESS BIOCHEMISTRY, ELSEVIER, NL, vol. 43, no. 3, 1 March 2008 (2008-03-01), pages 251-257, XP022611097, ISSN: 1359-5113, DOI: 10.1016/J.PROCBIO.2007.12.002 [retrieved on 2007-12-17]

### SEQUENCE LISTING

<110> ALKALOIDS CORPORATION
<120> PROCESS FOR THE CONVERSION OF COLCHICINOIDS TO THEIR 3-GLYCOSYLATED DERIVATIVES VIA THEIR RESPECTIVE 3-DEMETHYL ANALOGUES
<130> COLALK-A1770
<140> PCT/IB14/060093
   <141> 2014-03-24
<160> 1
<170> PatentIn version 3.5
<210> 1
   <211> 1445
   <212> DNA
   <213> Bacillus aryabhattai
<400> 1
   cagtcgagcg aactgattag aagcttgctt ctatgacgtt agcggcggac gggtgagtaa      60
   cacgtgggca acctgcctgt aagactggga taacttcggg aaaccgaagc taataccgga     120
   taggatcttc tccttcatgg gagatgattg aaagatggtt tcggctatca cttacagatg     180
   ggcccgcggt gcattagcta gttggtgagg taacggctca ccaaggcaac acgatgcata     240
   gccgacctga gagggtgatc ggccacactg ggactgagac acggcccaga ctcctacggg     300
   aggcagcagt agggaatctt ccgcaatgga cgaaagtctg acggagcaac gccgcgtgag     360
   tgatgaaggc tttcgggtcg taaaactctg ttgttaggga agaacaagta cgagagtaac     420
   tgctcgtacc ttgacggtac ctaaccagaa agccacggct aactacgtgc cagcagccgc     480
   ggtaatacgt aggtggcaag cgttatccgg ttattgggcg taaagcgcgc gcaggcggtt     540
   tcttaagtct gatgtgaaag cccacggctc aaccgtggag ggtcattgga aactggggaa     600
   cttgagtgca gaagagaaaa gcggaattcc acgtgtagcg gtgaaatgcg tagagatgtg     660
   gaggaacacc agtggcgaaa gcggcttttt ggtctgtaac tgacgctgag gcgcgaaagc     720
   gtggggagca aacaggatta gataccctgg tagtccacgc cgtaaacgat gagtgctaag     780
   tgttagaggg tttccgccct ttagtgctgc agctaacgca ttaagcactc cgcctgggga     840
   gtacggtcgc aagactgaaa ctcaaaggaa ttgacggggg cccgcacaag cggtggagca     900
   tgtggtttaa ttcgaagcaa cgcgaagaac cttaccaggt cttgacatcc tctgacaact     960
   ctagagatag agcgttcccc ttcgggggac agagtgacag gtggtgcatg gttctcgtca    1020
   gctcgtgtcg tgagatgttg ggttaagtcc cgcaacgagc gcaacccttg atcttagttg    1080
   ccagcattta gttgggcact ctaaggtgac tgccggtgac aaaccggagg aaggtgggga    1140
   tgacgtcaaa tcatcatgcc ccttatgacc tgggctacac acgtgctaca atggatggta    1200
   caaagggctg caagaccgcg aggtcaagcc aatcccataa aaccattctc agttcggatt    1260
   gtaggctgca actcgcctac atgaagctgg aatcgctagt aatcgcggat cagcatgccg    1320
   cggtgaatac gttcccgggc cttgtacaca ccgcccgtca caccacgaga gtttgtaaca    1380
   cccgaagtcg gtggagtaac cgtaaggagc tagccgccta aggtgggaca gatgattggg    1440
   gtgaa                                                                1445

## Description

### Field of the Invention

The present invention relates to a process for the preparation of glycosylated Colchicine, Thiocolchicine and their analogs and derivatives of formula (I). More particularly relates to the transformation of colchicines its analogs and derivatives into corresponding 3-glycosylated colchicine and the thio- analogs. 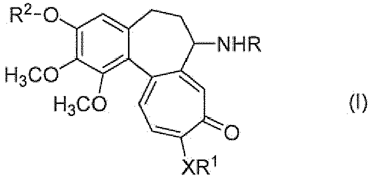 wherein X represents O or S, and R¹ represents C₁-C₆ alkyl, R² is a glycoside residue, R represents hydrogen, C₁-C₆ alkyl, formyl or acetyl.

The present invention relates to the process wherein a strain of the bacterial microorganism *Bacillus aryabhattai* with the accession number MTC 5875 is used for the bio-conversion.

### Background of the Invention

Colchicine is a medication that treats gout. It is a toxic natural product and secondary metabolite, originally extracted from plants of the family *Colchicaceae.* Currently, most of the Colchicine produced in the world is isolated from the seeds of the plant *Gloriosa superba.* Colchicine is an oral drug used for treating acute gout and familial Mediterranean fever (FMF). 3-demethyl-thiocolchicine glucoside i.e., Thiocolchicoside is a muscle relaxant which is known to possess GABA-mimetic and glycinergic actions. It is used as a muscle relaxant and anti-inflammatory drug in various countries including India. 3-demethylcolchicine glucoside or Colchicoside is an important intermediate which is used industrially for the production of Thiocolchicoside. Due to an increasing demand for Thiocolchicoside and limited use of Colchicine, an efficient method for the glycosidation of Colchicine-skeleton molecules to facilitate the preparation of both known compounds and novel derivatives for use in pharmacological research, would be very beneficial.

A number of efforts for the preparation of 3-glycosylcolchicine compounds have been carried out in the past, either by means of chemical reactions or by biotransformation.

FR 2112131 disclosed the glycosylation of thiocolchicine by reacting acetobromoglucose (or 2,3,4,6-tetra-o-acetyl)-α-D-glycopyranosyl bromide) with 2-demethyl-colchicine or 3-demethyl-thiocolchicine.

US patent 5,777,136 disclosed a chemical synthesis of colchicoside and thiocolchicoside by glycosylation. This process involves use of hazardous reagents such as sodium fluoride, boron trifluoride and pyridine for the glycosylation.

GB 923421A disclosed a process for the preparation of different derivatives of Colchicine by biotransformation using different microorganisms selected from Steptomyces, Fusarium, Saccharomyces, Curvularia, Corynebacterium, Cunnighamella, Aspergillus and Streptococcus.

The PCT publications WO 98/15642 A1 and WO 2005/108595 A disclosed a process for the preparation of 3-O-glycosylcolchicinoid compounds of formula (I) 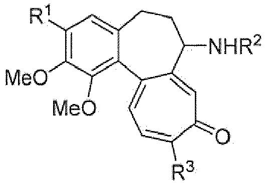 wherein R¹ is an O-glycoside residue, R² is hydrogen or C₁-C₇ acetyl, R³ is C₁-C₆ alkoxy or C₁-C₆ thioalkyl, comprising the biotransformation of compounds in which R¹ is OH or methoxy using *Bacillus megaterium,* characterised in that demethylated colchicinoids are used to induce the glycosylating enzyme system. The process disclosed in WO 98/156421 A1 gives the conversion product in a yield of 50-80%.

WO 99/18229 A1 disclosed a process for the preparation of 3-O-glycosylcolchicone compounds 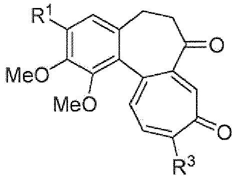 wherein R¹ is a glycoside residue, R³ is C₁-C₆ alkoxy or C₁-C₆ thioalkyl, which comprises the biotransformation of compounds in which R¹ is OH or methoxy by means of *Bacillus megaterium.*

WO 2012/038982 A2 disclosed the use of a gram-negative bacterial strain, *Providencia vermicola* for bio-transformation of thiocolchicine (TCN) to the corresponding 3-O-glycosyl derivative. The specific strain of *Providencia vermicola* was used for microbial transformation of other colchicinoid compounds such as Colchicine, thiocolchicine, 3-demethylcolchicine, 3-demethylthiocolchicine and N-deacetylthiocolchicine to their corresponding glycosylated form.

The Journal of Phytochemistry 1993, 33(4) 817-820 disclosed the glycosylation of thiocolchicine by a cell suspension culture of *Centella asiatica.*

The above said prior art methods have the drawbacks of excessive consumption of raw material during biotransformation and purification of the final glycosylated product. Hence, there is a need for a simple and industrially viable cost effective process for the glycosylation of colchicinoids.

Bacillus aryabhattai is a new species of microorganism which has been isolated, identified and characterised for the first time by Shivaji *et al* in 2009 from air samples collected from the upper atmosphere using cryotubes. There are several mutants that are reported in the literature or deposited with different culture collection centres in different countries. (Viz., VTCC - B-1092; VTCC-B-1097).

Indian Journal of Experimental Biology 51, April 2013, pp 322-335 disclosed a statistical and evolutionary optimization for enhanced production of an anti-leukemic enzyme, L-asparaginase, in a protease-deficient *Bacillus aryabhattai.*

Journal of Chemical Technology and Biotechnology DOI: 10.1002/jctb.4121 17 JUN 2013 disclosed a performance improvement of *Bacillus aryabhattai* ITBHU02 for high-throughput production of a tumor-inhibitory L-asparaginase using a kinetic model based approach.

Food Science and Technology, Jiangnan University, Wuxi 214122, China isolated novel strain of *Bacillus aryabhattai SK 22.003* from the soil of a dairy farm and it appeared to have higher β-galactosidase (EC 3.2.1.23) activity, which can be used to biosynthesize Galactooligosaccharides (GOS) from lactose. The objectives of this study were to investigate the characteristics of the β-galactosidase and the conditions influencing the biosynthesis of GOS.

WO 2011/112161 describe extended release pharmaceutical composition that contain 8 to 16 mg thiocolchicosiede. Furhtermore, WO 2011/112161 describe the muscle relaxing effect of thiocolchicoside and its specific gamma-aminobutiric acid (GABA) receptor antagonist action.

Bioscience Discovery 2012, 3(1), pp. 138-145 discloses that *Bacillus aryabhattai* is a dweller of the East Kolkatta Wetlands and that it is closely related to *Bacillus megaterium.*

Process Biochemistry 2008, 43, pp. 251-257 discloses that different Bacillus megaterium strains have different colchicine transforming activities.

The present inventors surprisingly found that *Bacillus aryabhattai* has the property to convert Colchicine and Thiocolchicine into their glycosylated compounds via their 3-demethyl analogues. Glycosylation of Colchicines using *Bacillus aryabhattai* has not been reported earlier and also in the above prior art.

### Objectives of the Invention

The main objective of the present invention is to provide a simple and cost effective bioprocess for the preparation of glycosylated Colchicine and Thiocolchicine, their analogs and derivatives of formula (I) which is selective.

In yet another objective of the present invention is to provide a process for the preparation of glycosylated Colchicine, its analogs and derivatives of formula (I) which is safe, industrially viable and eco-friendly.

Another objective is to provide a novel biotransformation method for easy, specific and quantitative production of the 3-O-glucosylcolchicinoid and 3-O-glucosylthiocolchicinoid compounds.

Another objective is to provide a novel biotransformation method for easy, specific and quantitative production of the 3-O-demethylcolchicinoid and 3-O-demethylthiocolchicinoid compounds.

### Summary of the Invention

The present invention relates to a bioprocess for the preparation of glycosylated Colchicine, its analogs and derivatives of formula (I) 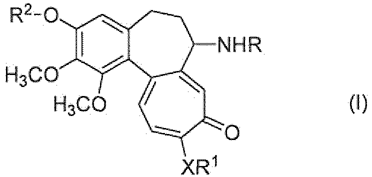 wherein X represents O or S, and R¹ represents C₁-C₆ alkyl, R² is a glycoside residue, R represents hydrogen, C₁-C₆ alkyl, formyl or acetyl, which comprises contacting colchicines, its analogs and derivatives of formula (II) 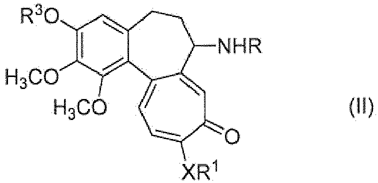 wherein X represents O or S, and R¹ represents C₁-C₆ alkyl, R³ represents hydrogen or methyl group, R represents hydrogen, C₁-C₆ alkyl, formyl or acetyl, with *Bacillus aryabhattai* in a suitable condition and isolating glycosylated colchicines, its analogs and derivatives of formula (I).

In a further embodiment, the bacterial microorganism of the species *Bacillus aryabhattai* for the aforementioned transformation is a strain (1011-6 AGM) isolated from soil and deposited under MTCC 5875 at The Microbial Type Culture Collection and Gene Bank (MTCC), Chandigarh, India. The strain has been accepted for deposit in MTCC-IDA under Budapest treaty and is preserved in the collection of MTCC.

### Detailed Description of the Invention

The word "Colchicine" or "Colchicinoid" described in the specification includes the Colchicine and all the Colchicine-skeleton molecules including colchicine, thiocolchicine, 3-O-demethylcolchicine (DMC) or 3-O-demethylthiocolchicine.

According to the present invention the strains of *Bacillus aryabhattai,* capable of growing in the presence of high concentrations of colchicine and thiocolchicine, have an exceedingly high, very specific biotransformation activity of converting colchicinoid substrates into glycosylated colchicinoid, its analogs and derivatives. Such a transformation is characterized by surprisingly high yields. Also, *Bacillus aryabhattai* is a gram positive organism and hence can be handled without any hazards.

The bio-transformation using *B.aryabhattai* proceeds via isolable 3-demethyl intermediates as depicted below:

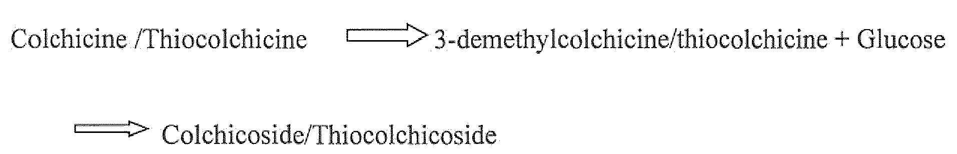

The chemical route for glycosylation consists of sequences of complex, nonspecific reactions which lead to a mixture of glycosylated derivatives, some of which are inactive. Therefore, yields of the product specifically glycosylated at C-3 of the aromatic ring, are very low. Surprisingly, the biotransformation of colchicine derivatives into glycosylcolchicinoid derivatives by means of *Bacillus aryabhattai* is selective (glycosylation exclusively at C-3 of the aromatic ring) and yields the product in the range of 90-99% of the theoretical yield.

In another embodiment the biotransformation is effected under a suitable condition needed for organism growth which includes time, pH and temperature. Accordingly the transformation is effected preferably at temperature of between about 15° C to about 55° C, at a pH level between 4 and 9.

In another embodiment we have found that bio-transformation using *Bacillus aryabhattai* proceeds via isolable intermediates namely the respective 3-demethyl analogues of Colchicine / Thiocolchicine which are produced selectively.

In another embodiment of the process we have found that the biotransformation takes place at a Colchicinoid concentration of 0.05 g/L to 3.0 g/L. The optimum concentration has been determined to be 1.0 to 1.5 g/L.

In the described process we have found that the biotransformation takes 18-40 hours for completion.

In another preferred embodiment, the present invention provides a biotransformation of Colchicinoids such as Colchicine, 3-demethylcolchicine, N-desacetyl-N-formylcolchicine, thiocolchicine and N-desacetyl-N-formylthiocolchicine to their glycosylated form by contacting with the organism *Bacillus aryabhattai* which possesses biocatalytic property to add glucose moiety to the Colchicinoid compounds.

In another embodiment, the present invention provides a process for the preparation of glycosylated derivatives of Colchicine and Thiocolchicine, wherein *Bacillus aryabhattai* is cultured in a liquid medium.

In another embodiment, the present invention provides a process for the preparation of glycosylated derivatives of Colchicine and Thiocolchicine, wherein *Bacillus aryabhattai* where in said medium contains one nitrogen source.

In another embodiment, the present invention provides a process for the preparation of glycosylated derivatives of Colchicine and Thiocolchicine, wherein said organic nitrogen source is selected from the group consisting of meat extract, peptone, tryptone, casein, hydrolysate, corn-step water.

In another embodiment, the present invention provides a process for the preparation of glycosylated derivatives of Colchicine and Thiocolchicine, wherein the medium contains at least one carbon source.

In another embodiment, the present invention provides a process for the preparation of glycosylated derivatives of Colchicine and Thiocolchicine, wherein the carbon source is selected from the group consisting of glucose, fructose, glycerol.

In another embodiment, the present invention provides a process for the preparation of glycosylated derivatives of Colchicine and Thiocolchicine, wherein the medium contains at least one source of inorganic salts of K⁺, Na⁺, Mg²⁺, NH₄⁺.

In another embodiment, glycosylated Colchicine prepared by the process of the present invention are used as a key intermediates for semi-synthetic production of the active drug substance Thiocolchicoside and also for preparing new derivatives.

The glycosylated Thiocolchicosine prepared by the process of the present invention can be used as a GABA-mimetic and glycinergic agent.

The glycosylated Thiocolchicosine prepared by the process of the present invention can be used as a muscle relaxant.

The organism *Bacillus aryabhattai* specifically converts the said alkaloids viz. Colchicine, Thiocolchicine and other related colchicinoid compounds to their 3-O-glycosyl derivatives when grown in AGM medium containing glucose as carbon source along with various other nutrients viz. Soya peptone, yeast extract, mineral salts.

In a preferred embodiment, the present invention provides a process for the preparation of glycosylated derivatives of Colchicine and Thiocolchicine, preferably, Colchicoside and Thiocolchicoside by contacting Colchicine and Thiocolchicine with *Bacillus aryabhattai.*

The gene sequence of the strain *Bacillus aryabhattai* MTCC 5875 is:

The glycosylated Colchicine derivatives obtained according to the claimed process were isolated from the fermented culture broths and subjected to various spectral analytical methods like IR, NMR, Mass Spectra and Optical rotation. The data obtained matched with that of the respective standards thus confirming the identity of the compounds viz. Colchicoside and Thiocolchicoside.

### Screening and Isolation of Bacteria:

Soil samples from different localities around Hyderabad, India were collected from agricultural fields, horticulture fields, botanical garden, Municipal Park, etc. in the following manner:
1. Wet soil samples about 10 - 20 gm around the root zone i.e. 6 - 10 inches from the surface were collected in sealable plastic bags.
2. The soil samples from the root zone of various horticultural, vegetable, medicinal and ornamental plants were collected from various locations and screened.

The soil samples thus collected were brought to the lab and stored in refrigerated conditions for further screening and bacterial isolation work.

The soil samples were screened for their physiological activity to convert Colchicine / Thiocolchicine to their respective glycosylated forms viz. Colchicoside / Thiocolchicoside. Throughout the screening studies we have used 1 - 2 gm of the collected soil samples; soil sample was transferred to 50 ml test tube having 10 ml of sterile distilled water; the soil suspension was mixed carefully to get uniform soil suspension. The soil suspension was allowed to stand for about 10 min and 1 ml of the supernatant from the soil suspension was transferred to 100 ml sterile LB broth (10 g peptone, 5 g yeast extract, 5 g sodium chloride in 1000 ml water) in 1 L conical flask with 100 mg of the substrate thiocolchicine and the flask was incubated on a rotary shaker at 30°C for 72 hr. A good mixed bacterial growth was observed after 72 hr of incubation; the broth was then diluted aseptically with 0.9% saline and plated on to LB agar medium having thiocolchicine in the concentrations ranging from 1.0 gm to 3.0 gm per lit. The LB plates were incubated at 30°C for 72 hr. The individual bacterial colonies were isolated separately on LB agar slants with Thiocolchicine. Likewise, many individual bacterial colonies were selected from various soil samples and re-inoculated in to LB broth with thiocolchicine. The broth samples were centrifuged in aseptic conditions and the supernatant was tested for the presence of Thiocolchicoside by TLC (Thin Layer Chromatography) as standard screening procedure for identifying potentially active bacteria with trans-glycosylation activity on thiocolchicine. By using the above mentioned screening procedure, we found one bacterium which showed strong trans-glycosylation activity and converted Colchicine to Colchicoside and Thiocolchicine to Thiocolchicoside. The isolated bacterium was further purified by "Single colony' isolation technique and found to have the following morphological characters.

### Bacterial Morphological characters:

The standard mature colonies show:
- Opaque and dull white colour
- Smooth circular colony with diameter 2 - 3 mm having smooth edge
- Centrally raised with entire margin
- Colonies are glistening and shiny.

The term "colony morphology" refers to the visible characteristics of a colony. Colonies that differ in appearance are typically different bacterial strains, species, or genera. However, colony morphology is not a reliable way to identify bacteria, as many different types of bacteria have similar colony morphology. Still, recognizing differences in colony morphology is useful when trying to isolate bacteria and also when plates of pure culture have become contaminated.

The isolated colony was stored in 20 % glycerol and stored at -20°C and the bacterial LB slants are stored at 4°C - 8°C. The slant culture was used for experimental purpose for media optimization. The isolated organism was sent to Microbial Type Culture Collection (MTCC), Indian Institute of Microbial Technology, Chandigarh, India for the purpose of identification. The organism was identified as *Bacillus aryabhattai.* To our surprise, this organism *Bacillus aryabhattai* has not been reported in the literature searched to have trans-glycosylation activity through which Thiocolchicine was converted to Thiocolchicoside and Colchicine to Colchicoside. The organism has been deposited with MTCC as per Budapest treaty on the International recognition of the deposit of microorganisms for the purposes of patent procedure and the MTCC accession number assigned is MTCC 5875.

The isolated organism was maintained on LB agar medium with Thiocolchicine and used for further studies.

### DESCRIPTION OF DRAWINGS:

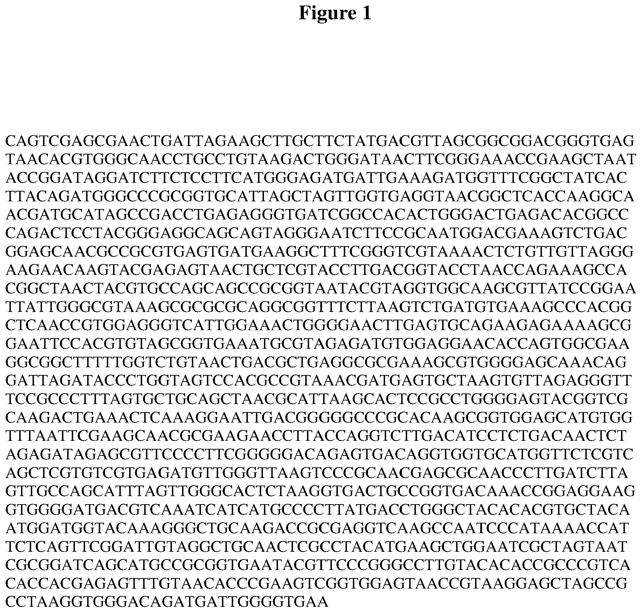

Figure 1: Sequence listing of gene sequence of the strain *Bacillus aryabhattai* MTCC 5875.

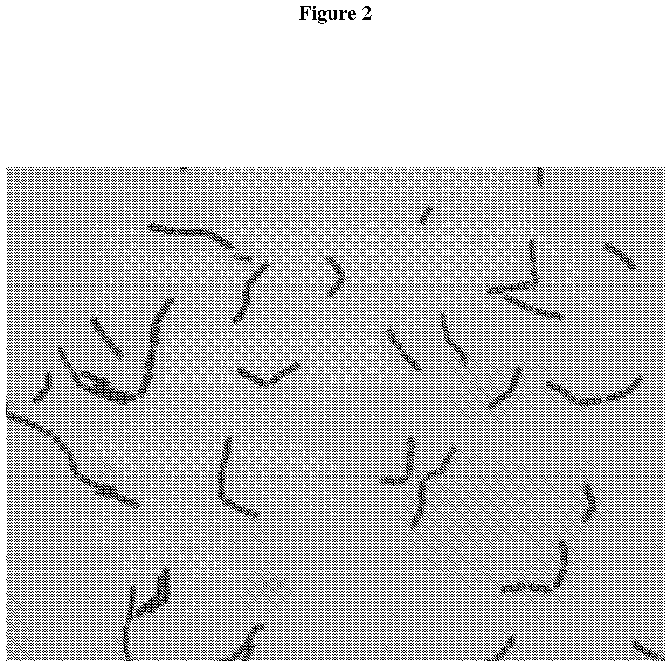

Figure 2: Photograph of *Bacillus aryabhattai* strain MTCC 5875

The following examples describe the nature of the invention and are given only for the purpose of illustrating the present invention in more detail and are not limited and relate to solutions which have been particularly effective on a bench scale.

### Reference-Example

### Isolation and screening of suitable bacteria having trans-glycosylation activity

Various bacteria having trans-glycosylation activity from Soil samples collected from various place in Jeedimetla, Hyderabad, Andhra Pradesh were screened using following media composition having pH 7.2 before sterilization.

About 1 gm of soil sample was taken and added to 10 ml of sterile water to make soil suspension and allowed to stand for about 10 min. About 1 ml of supernatant was added to 100 ml of sterile LB broth (10 g peptone, 5 g yeast extract, 5 g sodium chloride in 1000 ml water) in 1 lit conical flask with 100 mg of Thiocolchicine. The conical flask was incubated on a shaker at 30°C for about 72 hr. A good bacterial growth was observed after 72 hr of incubation; the broth was diluted aseptically with 0.9% saline and plated on to LB agar medium containing Thiocolchicine in the concentration of 1 g/L and the plates were incubated at 30°C in an incubator for about 72 hr. The colonies were isolated separately on LB agar slants with Thiocolchicine. Likewise, many bacterial colonies were isolated from soil samples (about 25 soil samples) collected from various locations as mentioned above. The broth samples were centrifuged in aseptic conditions and the supernatant was tested for the presence of the Thiocolchicoside by Thin Layer Chromatography as a standard procedure for screening the potentially active bacteria having trans-glycosylation enzyme/s. In total about 65 different isolates were screened for the trans-glycosylation activity on Thiocolchicine. During the screening process we isolated about five bacteria which are having different morphological characters and showing mild to strong trans-glycosylation activity. Out of this, one organism showed strong trans-glycosylation activity and converted Thiocolchicine to Thiocolchicoside and Colchicine to Colchicoside. Thus, the isolated bacteria were further tested on the following medium for the confirmation of trans-glycosylation activity.

| **S.No** | **Ingredients** | **g/Lit** |
|---|---|---|
| 1 | Peptone/Soya Peptone | 12 |
| 2 | Yeast Extract | 8 |
| 3 | NaCl | 9 |
| 4 | Thiocolchicine/Colchicine | 1 |
| 5 | Glucose | 20 |
| 6 | Agar | 14 |

### Example-1

### Formation of Thiocolchicoside or Colchicoside by bio-catalytic activity of bacterium Bacillus aryabhattai.

To explore and identify the trans-glycosylation activity of the bacterium *Bacillus aryabhattai,* the organism was maintained as Working Cell Bank (WCB) in 20% glycerol at -20°C; one WCB vial containing 500 µl of *Bacillus aryabhattai* glycerol suspension was transferred to a 500 ml conical flask containing 50 ml of the following seed medium :

### Seed medium:

| **S.No** | **Ingredients** | **g/Lit** |
|---|---|---|
| 1 | Peptone/Soya peptone | 8 |
| 2 | Yeast Extract | 6 |
| 3 | NaCl | 9 |
| 4 | Thiocolchicine/Colchinine | 0.2 |
| 5 | Glucose | 10 |

The above medium was sterilized (without Colchicine/Thiocolchicine) at 121°C for 20 min and 500 µl of glycerol suspension from one WCB vial was transferred to the medium (50 ml in 500 ml flask) and then the flask was incubated on a rotary shaker (50 mm through) with 175 RPM at 30°C for 22 - 24 hr till the OD at 550 nm (optical density) reaches 10 - 15. Thereafter, 5 - 8 % of the seed from the flask was transferred to the production medium with the following composition:

### Production Medium:

| **S.No** | **Ingredients** | **g/Lit** |
|---|---|---|
| 1 | Peptone/Soya Peptone | 12 |
| 2 | Yeast Extract | 8 |
| 3 | NaCl | 9 |
| 4 | Thiocolchicine/Colchicine | 1 |
| 5 | Glucose | 20 |
| 6 | pH | 7.2 |

After inoculation, the production flask was kept on rotary shaker with 175 RPM at 30°C for 36 hr; after 24 hr of incubation the samples were withdrawn and tested for the presence of Thiocolchicoside or Colchicoside by Thin Layer Chromatography (TLC). TLC was performed by applying 10 or 20 µl samples on Silica gel TLC F₂₅₄ with mobile phase {Ethyl Acetate: Ethanol: Water (7 : 2 : 1)}.

### Example-2

### Isolation of Colchicoside /Thiocolchicoside from the fermented broth

Two volumes of Methanol was added to the fermented broth and the precipitated matter was filtered through a bed of filter aid. The clear filtrate was concentrated to strip off the Methanol. The aqueous phase was partitioned successively with a mixture of Chloroform and Ethanol. The Thiocolchicoside/Colchicoside was extracted into the organic phase. The organic phase was concentrated in a rotary evaporator till all the solvent is removed. The residue was dissolved in a minimum quantity of hot Ethanol and allowed to crystallize at 4 to 8 °C for 8 hours. The crystallized product (Thiocolchicoside/Colchicoside) was filtered and dried in a vacuum oven to obtain the product in powder form.

The Thiocolchicoside / Colchicoside thus obtained was subjected to the following tests along with the respective standards:
a) Purity and assay by HPLC b)Specific Optical Rotation c)IR spectroscopy
d) ¹H NMR spectroscopy e) ¹³C NMR spectroscopy f) ESI Mass spectroscopy.

### Spectral data of Thiocolchicoside isolated from the fermented broth :

**¹H-NMR (300 MHz, DMSO-d₆) δ (ppm):** 1.83(3H-17,s); 1.9-2.3 (4H-5a,5b,6a&6b,m);2.40 (3H-18,m); 3.17-3.34 (6H-Glucoside protons 2',3',4',5'& 6',m); 3.52 (3H-13,s); 3.82 (3H-14,s); 4.27-4.37 (1H-7, m); 4.92 (1H-1',d); 7.00 (1H-8,s); 7.14 (1H-12,d); 7.25 (1H-11,d); 8.61 (NH-d).
**13C-NMR (300 MHz, DMSO-d₆) δ (ppm)** : 181.2 (C-9); 168.6 (C-16); 157.4 (C-10);151.1 (C-3 and C-7a - overlapping signal); 150.4 (C-1); 141.2 (C-2); 137.4 (C-12a); 134.0 (C-12); 127.8 (C-11); 126.6 (C-8); 126.5 (C-13); 111.1 (C-4);100.4 (C-1'); 77.2 (C-3'); 76.9 (C-5'); 73.3 (C-2'); 69.9 (C-4'); 60.9 (C-14); 60.7 (C-6'); 35.6 (C-6); 29.2 (C-5); 22.4 (C-17); 14.4(C-18)
**MS⁺(m/z)** : 564.18 [M + H]⁺
**Specific Optical Rotation : [α]_{D}²³ : -565.9° (c= 0.50, water)**

### Spectral data of Colchicoside isolated from the fermented broth :

**¹H-NMR (300 MHz,DMSO-d₆) δ (ppm):** 1.83(3H-17,s); 1.95-2.23 (4H-5a,5b,6a&6b,m); 3.14-3.72 (6H-Glucoside protons 2',3',4',5'& 6',m); 3.51 (3H-13,s); 3,82 (3H-14,s);3.86 (3H-18); 4.26-4.36 (1H-7, m); 4.90 (1H-1',d);6.99 (1H-8,s); 7.11 (1H-11,d); 7.06 (1H-12,d); 8.58 (NH-d).
**¹³C-NMR (300 MHz, DMSO-d₆) δ (ppm)** : 178.0 (C-9); 168.6 (C-16); 163.6 (C-10);150.8/150.9 (C-3 and C-7a - interchangeable signals); 150.4 (C-1); 141.2 (C-2); 135.1 (C-12a); 134.5 (C-12); 126.6 (C-8,C-11 -overlapping signals); 111.0 (C-4); 100.4 (C-1'); 77.2 (C-3'); 76.9 (C-5'); 73.3 (C-2'); 69.9 (C-4'); 60.9 (C-14); 60.7 (C-13); 60.8 (C-6'); 35.6 (C-6); 29.3 (C-5); 22.5 (C-17).
**MS⁺(m/z)** : 548.21 [M + H]⁺
**Specific Optical Rotation : [α]_{D}²² : -352.4° (c= 0.568, water)**

It was found that the spectral and other experimental data of both Colchicoside and Thiocolchicoside produced by fermentation route matches with that of the respective working standards that were isolated from plant sources and prepared semi-synthetically from Colchicoside respectively. The HPLC purity was found to be more than 99% for both Colchicoside and Thiocolchicoside.

### Example-3

### Formation of 3-demethylthiocolchicine or 3-demethylcolchicine by bio-catalytic activity of bacterium B. aryabhattai

By altering the media composition and process parameters viz. glucose concentration and pH described in Example-2, the organism converts the substrate to the intermediate which gets accumulated in the broth. The intermediates namely 3-demethylcolchicine and 3-demethylthiocolchicine were isolated in pure form from the broth and characterised by spectral methods.

### Spectral data of 3-demethylthiocolchicine isolated from the fermented broth :

**¹H-NMR (300 MHz,DMSO-d₆) δ (ppm):** 1.83(3H-17,s); 1.96-2.18 (4H-5a,5b,6a&6b,m);2.39 (3H-18,m); 3.51 (3H-13,s); 3.78 (3H-14,s); 4.29-4.38 (1H-7, m);7.00(1H-8,s); 6.52 (1H-4,s) 7.13 (1H-12,d); 7.24 (1H-11,d); 8.58 (NH-d).
**¹³C-NMR (300 MHz, DMSO-d₆) δ (ppm):** 181.1 (C-9); 168.5 (C-16); 156.9 (C-10); 151.3 (C-3); 151.1 (C-7a); 150.7 (C-1); 139.6 (C-2); 137.9(C-12a); 134.2(C-4a,12); 133.9(C-11); 126.6 (C-8); 123.8 (C-13); 111.3 (C-4); 60.7 (C-15); 60.5(C-14); 35.7(C-6);28.9(C-5);22.4(C-17);14.3(C-18)
**EI MS (m/z) :** M⁺ = 401

### Spectral data of 3-demethylcolchicine isolated from the fermented broth :

**¹H-NMR (300 MHz,CDCl₃) δ (ppm):** 1.98(3H-17,s); 2.24-2.49 (4H-5a,5b,6a&6b,m); 3.65 (3H-13,s) 3.99 (3H-2,s); 4.01 (3H-18,s); 4.61-4.68 (1H-7, m);; 6.58 (1H-4,s); 6.90 (1H-11,d); 7.34 (1H-12,d); 7.59(1H-8,s); 8.01 (NH-d).
**¹³C-NMR (300 MHz, CDCl₃) δ (ppm)** : 179.5 (C-9); 170.3 (C-16); 163.9 (C-10); 152.8 (C-7a); 150.2 (C-1); 149.9 (C-3); 139.2 (C-2); 137.1(C-12a); 135.5(C-12); 134.7(C-4a); 130.3 (C-8); 124.7 (C-12b);113.0(C-11);110.3(C-4); 61.3 (C-14); 61.2(C-13); 56.4(C-18);52.8(C-7);36.2 (C-6);29.4(C-5); 22.6(C-17)
**EI MS** : M⁺ = 385

The characterisation study proved that the intermediates were the 3-demethyl analogues namely 3-demethylcolchicine and 3-demethylthiocolchicine.

## Claims

1. A bioprocess for the preparation of glycosylated Colchicine, its analogs and derivatives of formula (I) 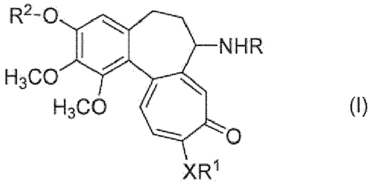 wherein the process is biotransformation, X represents O or S, and R¹ represents C₁-C₆ alkyl, R² is a glycoside residue, R represents hydrogen, C₁-C₆ alkyl, formyl or acetyl, which comprises contacting colchicines, its analogs and derivatives of formula (II) 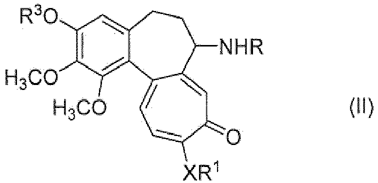 wherein X represents O or S, and R¹ represents C₁-C₆ alkyl, R³ represents hydrogen or methyl group, R represents hydrogen, C₁-C₆ alkyl, formyl or acetyl, with *Bacillus aryabhattai* in a suitable condition and isolating glycosylated colchicines, its analogs and derivatives of formula (I), wherein the biotransformation is carried using a microorganism of the species *Bacillus aryabhattai,* a gram positive bacterium with accession number MTCC 5875.

2. The process according to claim 1, wherein the biotransformation is high yielding and very specific in converting the Colchicinoids to their 3-glycosylated derivatives through their 3-demethyl analogues.

3. The process according to claim 1, wherein the bio-transformation using *Bacillus aryabhattai* with accession number MTCC 5875 proceeds selectively through respective 3-demethyl intermediates.

4. The process according to claim 3, wherein 3-demethyl intermediates formed after bio-transformation using *Bacillus aryabhattai* are isolated and converted into glycosylated Colchicines.

5. The process according to claim 3, wherein 3-demethyl intermediates formed after bio-transformation using *Bacillus aryabhattai* are converted *in situ* into glycosylated Colchicines.

6. The process according to claim 1, wherein the concentration of the Colchicinoid compound varies between 0.05 g/L to 3.0 g/L and more suitably 1.0 to 1.5 g/L.

7. The process according to claim 1, wherein the bio-transformation is completed within 18-40 hours.

8. The process according to claim 3 wherein demethylated intermediates are selected from 3-O-demethylcolchicine (DMC) or 3-O-demethylthiocolchicine (DMTC).

9. The process according to claim 1, wherein the transformation is effected at temperature of between 15° C to 55° C and pH level between 4 and 9.

10. The process according to claim 1, wherein *Bacillus aryabhattai* is cultured in a liquid medium and the medium contains one nitrogen source and nitrogen source is selected from the group consisting of meat extract, peptone, tryptone, casein, hydrolysate, corn-step water.

11. The process according to claim 10, wherein the medium contains at least one carbon source, which is selected from the group consisting of glucose, fructose, glycerol and at least one source of inorganic salts of K⁺, Na⁺, Mg²⁺, NH₄⁺.

12. The process of claim 1 further comprising the conversion of the glycosylated colchicines, analogs and derivatives into the active drug substance thiocolchicoside using a semi-synthetic procedure.

## Patentansprüche

1. Ein biologisches Verfahren zur Herstellung von glykolisierten Colchicin, seinen Analogen und Derivaten der Formel (I) 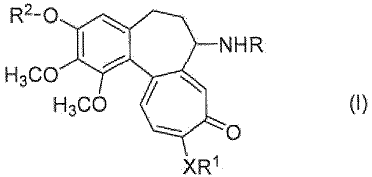 wobei das Verfahren eine Biotransformation ist, X O oder S darstellt und R¹ C₁-C₆ Alkyl darstellt, R² ein Glykosidrest ist, R stellt Wasserstoff, C₁-C₆ Alkyl, Formyl oder Acetyl dar, welches das in Kontakt bringen des Colchicins, seiner Analoge und Derivate der Formel (II) 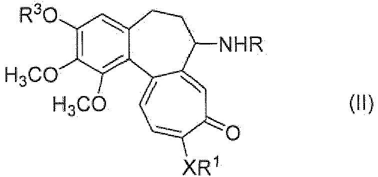 worin X O oder S darstellt, und R¹ C₁-C₆ Alkyl darstellt, R³ Wasserstoff oder Methyl darstellt, R Wasserstoff, C₁-C₆ Alkyl, Formyl oder Acetyl darstellt, mit *Bacillus aryabhattai* unter geeigneten Bedingungen und isolieren des glykolisierten Colchicins, seiner Analoge und Derivate der Formel (I), wobei die Biotransformation unter Verwendung eines Mikroorganismus der Spezies *Bacillus aryabhattai,* einem gram-positiven Bakterium mit der Zugangsnummer MTCC 5875, durchgeführt wird.

2. Das Verfahren nach Anspruch 1, wobei die Biotransformation mit hoher Ausbeute erfolgt und sehr spezifisch hinsichtlich der Umwandlung der Colchicinoide in ihre 3-glykolisierten Derivate durch ihre 3-demethylierten Analoge ist.

3. Das Verfahren nach Anspruch 1, wobei die Bio-Transformation unter Verwendung von *Bacillus aryabhattai* mit der Zugangsnummer MTCC 5875 selektiv durch die entsprechenden 3-demethylierten Intermediate voranschreitet.

4. Das Verfahren nach Anspruch 3, wobei die nach der mit *Bacillus aryabhattai* durchgeführten Bio-Transformation gebildeten 3-demethylierten Intermediate isoliert werden und in glykolisierte Colchicine konvertiert werden.

5. Das Verfahren nach Anspruch 3, wobei die nach der mit *Bacillus aryabhattai* durchgeführten Bio-Transformation gebildeten 3-demethylierten Intermediate *in situ* in glykolisierte Colchicine konvertiert werden.

6. Das Verfahren nach Anspruch 1, wobei die Konzentration der Colchicinoid-Verbindung zwischen 0.05 g/L bis 3.0 g/L variiert, geeigneter zwischen 1.0 bis 1.5 g/L.

7. Das Verfahren nach Anspruch 1, wobei die Bio-Transformation innerhalb von 18-140 Stunden abgeschlossen ist.

8. Das Verfahren nach Anspruch 3, wobei die demethylierten Intermediate ausgewählt werden aus 3-O-Demethylcolchicin (DMC) oder 3-O-demethylthiocolchicin (DMTC).

9. Das Verfahren nach Anspruch 1, wobei die Transformation bei einer Temperatur zwischen 15° C bis 55° C und einem pH-Wert zwischen 4 und 9 ausgeführt wird.

10. Das Verfahren nach Anspruch 1, wobei *Bacillus aryabhattai* in einem flüssigen Medium kultiviert wird und das Medium eine Stickstoffquelle enthält und die Stickstoffquelle ausgewählt ist aus Fleischextrakt, Pepton, Trypton, Casein, Hydrolysat, Maisquellwasser.

11. Das Verfahren nach Anspruch 10, wobei das Medium wenigstens eine Kohlenstoffquelle enthält, welche aus der Gruppe bestehend aus Glukose, Fruktose, Glycerin ausgewählt wird und wenigstens eine Quelle eines anorganischen Salzes von K⁺, Na⁺, Mg²⁺, NH₄⁺.

12. Das Verfahren nach Anspruch 1 weiterhin umfassend die Umwandlung der glykolisierten Colchicine, Analoge und Derivate in die aktive Arzneimittelsubstanz Thiocolchicid unter Verwendung eines halbsynthetischen Verfahrens.

## Revendications

1. Processus biologique pour la préparation de Colchicine glycosylée, ses analogues et dérivés de la formule (I) 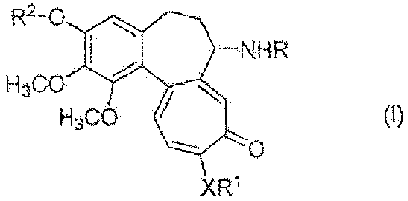 où le processus est une biotransformation, X représente O ou S, et R¹ représente un alkyle en C₁-C₆, R² est un reste glycoside, R représente un atome d'hydrogène, un alkyle en C₁-C₆, un formyle ou un acétyle, qui comprend la mise en contact de colchicines, leurs analogues et dérivés de formule (II) 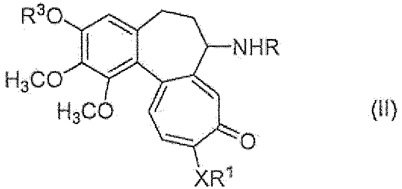 où X représente O ou S, et R¹ représente un groupe alkyle en C₁-C₆, R³ représente un groupe hydrogène ou méthyle, R représente un atome d'hydrogène, un alkyle en C₁-C₆, un formyle ou un acétyle, avec *Bacillus aryabhattai* dans un état approprié, et l'isolation de colchicines glycosylées, leurs analogues et dérivés de formule (I), dans lequel la biotransformation est réalisée en utilisant un microorganisme de l'espèce *Bacillus aryabhattai,* une bactérie à Gram positif portant le numéro d'accès MTCC 5875.

2. Processus selon la revendication 1, dans lequel la biotransformation est d'un rendement élevé et très spécifique pour convertir les Colchicinoides en leurs dérivés 3-glycosylés par leurs analogues de 3-déméthyle.

3. Processus selon la revendication 1, dans lequel la biotransformation en utilisant *Bacillus aryabhattai* avec le numéro d'accès MICC 5875 passe sélectivement par des intermédiaires de 3-déméthyle respectifs.

4. Processus selon la revendication 3, dans lequel des intermédiaires de 3-déméthyle formés après la biotransformation en utilisant *Bacillus aryabhattai* sont isolés et convertis en Colchicines glycosylées.

5. Processus selon la revendication 3, dans lequel des intermédiaires de 3-déméthyle formés après biotransformation en utilisant *Bacillus aryabhattai* sont convertis *in situ* en Colchicines glycosylées.

6. Processus selon la revendication 1, dans lequel la concentration du composé colchicinoide varie entre 0,05 g/L et 3,0 g/L, et de manière plus adaptée entre 1,0 et 1,5 g/L.

7. Processus selon la revendication 1, dans lequel la biotransformation est achevée en 18 à 40 heures.

8. Processus selon la revendication 3, dans lequel des intermédiaires de 3-déméthyle sont choisis parmi 3-O-déméthylcolchicine (DMC) ou 3-O-deméthylthiocolchicine (DMTC).

9. Processus selon la revendication 1, dans lequel la transformation est effectuée à une température comprise entre 15°C et 55°C et à un niveau de pH compris entre 4 et 9.

10. Processus selon la revendication 1, dans lequel *Bacillus aryabhattai* est cultivée dans un milieu liquide et le milieu contient une source d'azote et la source d'azote est choisie dans le groupe constitué d'un extrait de viande, de peptone, de tryptone, de caséine, d'hydrolysat, et d'eau d'étape de maïs.

11. Processus selon la revendication 10, dans lequel le milieu contient au moins une source de carbone, qui est sélectionnée dans le groupe constitué de glucose, fructose, glycérol et au moins une source de sels inorganiques de K⁺, Na⁺, Mg²⁺, NH₄⁺.

12. Processus selon la revendication 1, comprenant en outre la conversion des colchicines glycosylées, analogues et dérivés en la substance médicamenteuse active thiocolchicoside en utilisant une procédure semi-synthétique.
